# EUROPÄISCHE PATENTSCHRIFT

(11) **EP 0 733 704 B1**
(45) Veröffentlichungstag und Bekanntmachung des Hinweises auf die Patenterteilung: **15.10.1997**
(21) Anmeldenummer: 96104475.7
(22) Anmeldetag: 21.03.1996
(51) Int. Cl.: C12N 15/11, C12P 21/00, C12N 1/11, C12N 1/21, C07K 14/00

(54) **Steuerbares Expressionssystem**
Controlable expression system
Système d'expression contrôlable

(30) Priorität: 24.03.1995 DE 19510930; 13.04.1995 DE 19514056
(43) Veröffentlichungstag der Anmeldung: 25.09.1996
(73) Patentinhaber: Consortium für elektrochemische Industrie GmbH, D-81379 München (DE)
(72) Erfinder: Böck, August, Prof. Dr., 82269 Geltendorf (DE); Mayer, Dagmar, 81673 München (DE); Schlensog, Verena, Dr., 85737 Ismaning (DE); Candussio, Anton, Dr., 81825 München (DE)
(74) Vertreter: Potten, Holger

(56) Entgegenhaltungen:
- JOURNAL OF BACTERIOLOGY, Bd. 177, Nr. 18, 1995, Seiten 5261-5269; D. MAYER ET AL.: "Identification of the transcriptional activator controlling the butanediol fermentation pathway in Klebsiella terrigena"
- JOURNAL OF BACTERIOLOGY, Bd. 175, Nr. 5, 1993, Seiten 1392-1404; K. BLOMQUIST ET AL.: "Characterization of the genes of the 2,3-butanediol operons from Klebsiella terrigena and Enterobacter aerogenes"

## Beschreibung

Die Erfindung betrifft ein steuerbares Expressionssystem, Verfahren zu seiner Herstellung und Verwendung.

Ein wichtiges Anwendungsgebiet der modernen Gentechnologie ist die Produktion definierter Proteine unter Verwendung rekombinanter Produktionssysteme. Solche rekombinanten Produktionssysteme bestehen mindestens aus zwei Komponenten, nämlich a) einem Wirt, der die zelluläre Maschinerie zur Proteinproduktion zur Verfügung stellt, und b) einer rekombinanten DNS, die für das zu produzierende Protein kodiert.

Bekannte Wirtssysteme sind z.B. Mikroorganismen, Tiere, Pflanzen oder eukaryotische Zellkulturen. Zur Produktion großer Mengen rekombinanter Proteine werden als Wirtssysteme vorzugsweise Mikroorganismen wie Pilze oder Bakterien, besonders bevorzugt E. coli, verwendet.

Die rekombinante DNS, die die genetische Information zur Produktion eines definierten Proteins enthält, kann in ein Chromosom des Wirts integriert sein oder episomal, in Form eines Plasmids, Cosmids oder Phagen, bzw. Virus vorliegen. Neben der genetischen Information für das zu produzierende Protein enthält die rekombinante DNS Regulationselemente, sog. Promotoren, die für den ersten Schritt der Expression von Strukturgenen, die Transkription der DNS - Sequenz in RNS, nötig sind. Promotoren, definierte kurze DNS - Regionen, dienen dabei als Erkennungsstellen für RNS - Polymerasen, Enzyme, die die Transkription von DNS - Sequenzen in RNS katalysieren.

Promotoren sind häufig funktionell kombiniert mit DNS - Regionen die als Erkennungs-, oder Bindestellen für eine Gruppe von Proteinen dienen, die in Abhängigkeit von unterschiedlichen Stimuli die Aktivität von Promotoren beeinflussen und daher als Regulatoren bezeichnet werden. Durch die Bindung solcher Regulatoren an ihre Bindestellen können damit verbundene Promotoren aktiviert (Aktivatoren) oder reprimiert (Repressoren) werden. In gentechnischen Systemen zur Produktion rekombinanter Proteine werden solche Regulator/Promotor - Interaktionen verwendet, um die Produktion von Zielproteinen zu steuern.

Beispiele für solche technisch verwendeten Systeme sind der lac- und tac-Promotor, bei denen es sich um Promotoren handelt, die durch Bindung des sog. lac-Repressorproteins inaktiviert werden. Bei Zugabe von Laktose oder Laktoseanaloga wie IPTG dissoziiert der Repressor von seiner Bindestelle ab; es kommt zu einer Induktion der Expression von distal zum Promotor gelegenen Genen.

Ein weiteres technisch verwendetes Regulationssystem besteht aus der Kombination trp-Promotor und trp-Repressorprotein. Nur in Anwesenheit von Tryptophan bindet der Repressor an den Promotor und inaktiviert diesen.

Für eine technische Produktion von rekombinanten Proteinen besitzen diese Systeme mehrere Nachteile. Die zur Induktion oder Repression dieser Systeme verwendeten Substanzen sind teuer und schwer handhabbar, besonders wenn es sich um metabolisierbare Substanzen wie Laktose oder Tryptophan handelt. Das molare Verhältnis von Regulatorprotein und Promotor beeinflußt stark die Reprimierbarkeit des Expressionssystems. Bei einem Überschuß des Promotors sind solche Systeme nicht vollständig reprimierbar, da der Repressor austitriert wird. Die vom lac - Repressor abhängigen Promotoren sind bei einem molaren Überschuß des Repressors darüber hinaus nicht vollständig induzierbar.

In DE 39 26 076 A1 (entspricht CA-A-2015046) wird die Verwendung des pfl-Promotors zur Produktion rekombinanter Proteine beschrieben. Dieser E. coli - eigene Promotor wird in Anwesenheit des Regulatorproteins FNR unter anaeroben Bedingungen und durch Pyruvat induziert und durch Sauerstoff reprimiert. Im Vergleich zum lac-, tac- oder trp-Promotor besitzt der pfl-Promotor den Vorteil, daß er technisch einfach und billig zu induzieren ist. Die Regulation erfolgt dadurch, daß zu Beginn der Fermentation die Aktivität des Promotors durch die Zufuhr von Sauerstoff unterdrückt wird. In der späten logarithmischen Wachstumsphase tritt durch die hohe Zellmasse automatisch eine Sauerstofflimitierung ein, die fermentationstechnisch verstärkt oder reguliert werden kann. Zusätzlich wird in dieser Wachstumsphase vom Wirtsorganismus Pyruvat gebildet, das die Expression verstärkt. Gegebenenfalls kann extern weiteres Pyruvat zur Steigerung der Induktion zugesetzt werden.

Trotz seiner Vorteile gegenüber den anderen Promotoren ist dieser Promotor aus folgenden Gründen nur bedingt zur technischen Produktion von rekombinanten Proteinen geeignet. Bei Verwendung des pfl - Promotors ist es nicht möglich, die Expression rekombinanter Proteine in Anwesenheit von Sauerstoff zu induzieren. Es ist jedoch wünschenswert, die maximale Syntheseleistung des Wirts, die bei E. coli bei aerobem Wachstum während der exponentiellen Wachstumsphase vorliegt, zur Produktion von rekombinanten Proteinen zu nutzen. Obwohl der pfl - Promotor unter aeroben Bedingungen nicht induzierbar ist, besitzt er in Anwesenheit von Sauerstoff eine Grundaktivität, d.h. der pfl - Promotor ist nicht vollständig reprimierbar. Die minimale Restaktivität des pfl - Promotors unter aeroben Bedingungen beträgt 5 - 10 % der Aktivität des Promotors unter optimalen Induktionsbedingungen (anaerob mit Pyruvat). Das Regulationssystem bestehend aus FNR - Regulatorprotein und pfl - Promotor ist damit ein System, das zwar technisch einfach und kostengünstig zu steuern ist, das jedoch nicht vollständig reprimierbar und nicht unter allen Wachstumsbedingungen induzierbar ist. Wie alle anderen bekannten technisch verwendeten Promotorsysteme ist damit auch der pfl - Promotor nur bedingt zur Expression von Strukturgenen oder rekombinanten Genen geeignet.

Von Blomquist et al. (J. Bact. (1993) Vol 175(5), S. 1392 - 1404) wurde die DNS - Sequenz des 2,3 - Butandiol Operons (bud - Operon) aus Klebsiella terrigena beschrieben. Es ist bekannt, daß in Klebsiella terrigena die Bildung von 2,3 - Butandiol durch einen niedrigen pH - Wert, die Gegenwart von Acetat und bei Sauerstofflimitierung induziert wird. Blomquist et al. zeigten, daß die Gene, die für die zur 2,3 - Butandiolbildung benötigten Proteine kodieren, ein Operon bilden, dessen Transkription durch eine Sauerstofflimitierung induziert wird. Die Literaturstelle enthält keine Angabe über eine Induktion des Promotors in Gegenwart von Sauerstoff.

Aufgabe der Erfindung war es, ein Expressionssystem zur Verfügung zu stellen, das eine technisch einfach zu steuernde Expression unter allen Wachstumsbedingungen des Wirtsorganismus erlaubt.

Gegenstand der Erfindung ist ein durch Acetat, pH-Wert und Sauerstoff steuerbares Expressionssystem, umfassend ein in trans wirkendes Regulatorprotein und einen von diesem Protein aktivierbaren Promotor, dadurch gekennzeichnet, daß das Regulatorprotein eine Aminosäuresequenz, die zu mindestens 75% homolog zu der Aminosäuresequenz Seq. ID-No:1 ist, umfaßt und der Promotor, eine DNS-Sequenz umfaßt, die zu mindestens 95% homolog zu den Basen 315 bis 397 der DNS-Sequenz Seq. ID-No:2 ist.

Das erfindungsgemäße Expressionssystem exprimiert beliebige Strukturgene unter Kontrolle des Expressionssystems bei einem Sauerstoffpartialdruck pO₂ von 0-5%, einem pH-Wert von 6,0-6,5 und in Gegenwart von Acetat in einer Konzentration von 40-60 mM maximal.

Das erfindungsgemäße Expressionssystem ermöglicht erstmals eine technisch auch im großen Maßstab einfach durchführbare, billige Regulation der Expression rekombinanter Genprodukte unabhängig von der Wachstumsphase der Produzentenstämme und dem O₂ - Gehalt des Anzuchtmediums.

Gegenstand der vorliegenden Erfindung ist ferner ein Regulatorprotein, welches dadurch gekennzeichnet ist, daß es bei Sauerstofflimitierung und Anwesenheit von Acetat und einem pH-Wert des Anzuchtmediums von pH 6,0 bis pH 6,5 eine optimale Aktivierung des bud - Promotors aus Klebsiella terrigena (DSM2687) bewirkt.

Unter Sauerstofflimitierung im oben verwendeten Sinn ist ein Partialdruck pO₂ von 0 - 5% zu verstehen. Die Acetat-Konzentration beträgt bei diesen Bedingungen vorzugsweise 40 - 60 mM.

Vorzugsweise umfaßt das erfindungsgemäße Regulatorprotein eine Aminosäuresequenz, die zu mindestens 75 % homolog zu der Aminosäuresequenz Seq ID-No: 1 ist.

In einer besonders bevorzugten Ausführungsform umfaßt die Aminosäuresequenz des erfindungsgemäßen Regulatorproteins die Aminosäuresequenz Seq ID-No: 1. Ein solches Regulatorprotein wird im Folgenden auch als BudR bezeichnet.

Der Promotor des Expressionssystems kann eine beliebige DNS - Region umfassen, die unter BudR aktivierenden Bedingungen zu einer BudR - abhängigen Initiation der Transkription der unmittelbar 'downstream' zu dieser Region gelegenen Gene führt. Zu den physiologischen Bedingungen, bei denen BudR maximal aktiviert wird, gehören ein Sauerstoffpartialdruck pO₂ von 0 - 5%, die Gegenwart von Acetat in einer Konzentration von 40 -60 mM und ein pH-Wert von 6,0 - 6,5.

Vorzugsweise umfaßt der Promotor eine DNS-Sequenz, die zu mindestens 95 % homolog zu den Basen 315 bis 456 in der DNS-Sequenz ID-No: 2 ist.

In einer besonders bevorzugten Ausführungsform umfaßt der Promotor die DNS - Sequenz ID-No: 2 im Bereich der Basen 315 bis 397. Eine solche DNS-Region wird im Folgenden auch als bud-Promotor bezeichnet.

Das Gen für das erfindungsgemäße Regulatorprotein kann entweder vollständig chemisch oder enzymatisch in vitro anhand der in Seq. ID-No: 1 offenbarten Sequenz synthetisiert werden oder es kann aus einem 2,3 - Butandiol - bildenden Mikroorganismus isoliert werden.

Das erfindungsgemäße Regulatorprotein erhält man vorzugsweise durch Expression eines derartig erhältlichen Gens. Die Erfindung betrifft daher ebenfalls Gene die für erfindungsgemäße Regulatorproteine kodieren.

Die Klonierung eines Gens, das für ein erfindungsgemäßes Regulatorprotein kodiert, erfolgt vorzugsweise unter Verwendung eines sogenannten Reporterstamms. Die Konstruktion von Reporterstämmen für Transkriptions-Aktivatoren ist dem Fachmann vertraut. Ein solcher Stamm enthält ein Gen für ein vorzugsweise einfach nachzuweisendes Protein unter der transkriptionellen Kontrolle des bud - Promotors. Ein Beispiel für einen Reporterstamm ist E.coli BL 142 (vergl. Beispiele 5 und 7).

Als Reportergen wird vorzugsweise das dem Fachmann bekannte Gen für eine β - Galaktosidase auf Plasmid pRS552 (Simons et al. (1987), Gene, Vol 53, p. 85 - 96 ) verwendet.

Zur Klonierung eines Gens, das für ein erfindungsgemäßes Regulatorprotein kodiert, wird in einen solchen Reporterstamm eine Genbank aus einem Mikroorganismus, der ein erfindungsgemäßes Regulatorprotein bildet, eingebracht. Die Konstruktion einer Genbank und deren Isolierung ist dem Fachmann ebenfalls vertraut.

Das Verfahren der Klonierung mittels Reporterstamm ist dem Fachmann auch bekannt. Es kann beispielsweise auf die in Casadaban & Cohen (1979) Proc.Natl.Acad.Sci.USA Vol.76, No.9, pp.4530-4533 beschriebene Weise erfolgen.

Zur Detektion von Klonen, die das gesuchte Protein bilden, werden aus der Genbank solche Klone isoliert, bei denen das Reportergen bei pH 6,5 und in Gegenwart von Acetat induziert wird. Im Falle einer Verwendung von β-Galaktosidase als Reportergen enthalten die Indikatorplatten beispielsweise den Farbstoff X-Gal. Solche Klone, die im Falle einer Verwendung von β-Galaktosidase als Reportergen auf X-Gal - haltigen Indikatorplatten tief dunkelblau werden, enthalten ein Gen für ein erfindungsgemäßes Regulatorprotein.

In einer bevorzugten Ausführungsform der Erfindung wird das Gen für das Regulatorprotein aus einer Genbank von Mikroorganismen der Gattungen Enterobacter oder Klebsiella, vorzugsweise aus Klebsiella terrigena (käuflich erhältlich bei der DSM-Deutschen Sammlung für Mikroorganismen und Zellkulturen GmbH, Mascheroder Weg 1b, D-38124 Braunschweig unter der Bezeichnung DSM2687), isoliert. Klebsiella terrigena ist bei der Internationalen Hinterlegungsstelle unter der genannten Anschrift unter der Eingangsnummer DSM 9883 gemäß Budapester Vertrag hinterlegt.

Grundsätzlich kann das Gen für das Regulatorprotein aus einem Mikroorganismus, beispielsweise aus einem 2,3-Butandiol bildenden Mikroorganismus, inbesondere aus einem 2,3-Butandiol bildenden Mikroorganismus aus der Familie der Enterobacteriaceae isoliert werden.

Verfahren zur Herstellung eines für das Expressionssystem geeigneten Promotors sind dem Fachmann geläufig und werden daher nicht im Detail erläutert. Ein solches Verfahren zur Herstellung eines DNS - Fragments, welches die Promotoraktivität gemäß der Erfindung vermittelt, ist die chemische oder enzymatische 'de novo' Synthese unter Verwendung der in Seq.ID.No.#2 angegebenen Sequenzinformation.

Ein weiteres Verfahren zur Herstellung des Promotors umfaßt die Modifikation eines beliebigen DNS - Fragments mittels dem Fachmann bekannter Mutagenesemethoden unter Verwendung der in Seq.ID.No.#2 angegebenen Sequenzinformation.

Ein weiteres Verfahren zur Herstellung des Promotors umfaßt die Isolation des Promotors aus einer Genbank eines 2,3 - Butandiol bildenden Mikroorganismus. 2,3-Butandiol bildende Mikroorganismen sind allgemein bekannt und der Öffentlichkeit zugänglich. Beispiele sind Vertreter der Gattungen Enterobacter, Serratia, Erwinia und Klebsiella. Bei diesem Verfahren wird zumindest die upstream - regulatorische Region des bud - Operons, ein 5' vor dem Strukturgen gelegener DNS-Bereich, der eine die Expression des Strukturgens regulierende Funktion besitzt, aus einem solchen Organismus in an sich bekannter Weise isoliert. Die mitisolierten Teile, die keine regulatorische Funktion besitzen, werden gegebenenfalls nach bekannten Methoden entfernt (vgl. Beispiel 14).

Vorzugsweise wird ein den Promotor enthaltendes DNS - Fragment aus einer Genbank eines Bakteriums der Gattung Enterobacter oder Klebsiella, besonders bevorzugt aus einer Genbank von Klebsiella terrigena (DSM2687) isoliert.

Ein weiterer Gegenstand der Erfindung ist eine Expressionskassette, die dadurch gekennzeichnet ist, daß ein für das Expressionssystem geeigneter Promotor funktionell mit dem Strukturgen eines zu exprimierenden, rekombinanten Proteins verknüpft ist.

In einer bevorzugten Ausführungsform der Expressionskassette liegt zwischen Promotorregion und zu exprimierendem Strukturgen ein transkribierter, aber untranslatierter Bereich, der eine Ribosomenbindestelle enthält.

Eine erfindungsgemäße Expressionskassette läßt sich beispielsweise dadurch herstellen, daß der Promotor in einer dem Fachmann bekannten Art und Weise vor das 5' - Ende eines zu exprimierenden Strukturgens kloniert wird.

Die Erfindung betrifft ferner Mikroorganismen, welche eine erfindungsgemäße Expressionskassette enthalten.

Die Expressionskassette kann dabei in das Genom des Mikroorganismus integriert sein, sie kann aber auch episomal auf mindestens einem Vektor vorliegen. Vorzugsweise liegt die Expressionskassette episomal auf mindestens einem Vektor vor.

In der erstgenannten Ausführungsform wird die erfindungsgemäße Expressionskassette mit bekannten Methoden in das Genom des Wirtsorganismus integriert.

In der als Zweites genannten, bevorzugten Ausführungsform liegt die Expressionskassette im Wirtsorganismus episomal auf einem Vektor, dem sogenannten Expressionsvektor vor. Die Integration der erfindungsgemäßen Expressionskasette in einen Vektor erfolgt nach den üblichen, dem Fachmann bekannten Methoden. Geeignete Vektormoleküle sind dem Fachmann bekannt. Ein Beispiel für einen solchen Vektor ist der dem Fachmann bekannte Vektor pJF118 (Fürste et al. (1986), Gene Vol 48, 119 - 131) und Derivate davon.

Die Erfindung betrifft ferner ein Verfahren zur Herstellung eines erfindungsgemäßen Expressionssystems, welches dadurch gekennzeichnet ist, daß in einen beliebigen Mikroorganismus ggf. mindestens ein Gen für ein erfindungsgemäßes Regulatorprotein und/oder ggf. mindestens eine erfindungsgemäße Expressionskassette eingebracht werden, so daß in dem jeweiligen Mikroorganismus anschließend mindestens ein Gen für ein erfindungsgemäßes Regulatorprotein und eine erfindungsgemäße Expressionskassette vorhanden sind.

Die Erfindung betrifft daher auch Mikroorganismen, die ein erfindungsgemäßes Expressionssystem enthalten.

Das erfindungsgemäße Expressionssystem läßt sich beispielsweise dadurch herstellen, daß in einen beliebigen Mikroorganismus, der mindestens ein Gen für ein erfindungsgemäßes Regulatorprotein enthält, eine erfindungsgemäße Expressionskassette eingebracht wird.

Vorzugsweise werden bei diesem Verfahren zur Herstellung des erfindungsgemäßen Expressionssystems solche Mikroorganismen verwendet, welche natürlicherweise ein Gen für ein erfindungsgemäßes Regulatorprotein enthalten.

Besonders bevorzugt werden bei diesem Verfahren Mikroorganismen der Gattungen Enterobacter oder Klebsiella, die ein Gen für ein erfindungsgemäßes Regulatorprotein enthalten.

Besonders bevorzugt wird in diesem Verfahren Klebsiella terrigena (DSM2687) verwendet.

Ferner werden bei diesem Verfahren besonders bevorzugt solche Mikroorganismen als Wirtsstämme für eine erfindungsgemäße Expressionskassette verwendet, denen ein Gen für ein erfindungsgemäßes Regulatorprotein in dem Fachmann bekannter Weise zugeführt worden ist. Das zugeführte Gen, das für das Regulatorprotein kodiert, kann dabei episomal vorliegen, oder in das Chromosom des Wirts integriert sein.

Liegt das zugeführte Gen, das für das Regulatorprotein kodiert, episomal vor, wird es vorzugsweise auf dem gleichen Vektormolekül lokalisiert sein, das auch die Expressionskassette trägt. Daneben ist aber auch eine Anordnung bevorzugt, bei der das zugeführte Gen für ein erfindungsgemäßes Regulatorprotein und die erfindungsgemäße Expressionskassette auf zwei verschiedenen, aber komplementären Vektormolekülen, wie sie dem Fachmann bekannt sind, gleichzeitig in einer Zelle des Wirtsorganismus vorliegen.

In einer allgemeinen Ausführungsform werden für die Konstruktion von Expressionssystemen, bei denen dem Wirtsstamm ein Gen, das für das Regulatorprotein kodiert, zusätzlich zugeführt worden ist, als Wirtsstämme gram - positive oder gram - negative Bakterien verwendet.

Bevorzugt werden als Wirtsstämme für ein solches Expressionssystem Bakterien der Familie Enterobacteriaceae, besonders bevorzugt solche der Gattungen Escherichia und Salmonella, verwendet. Besonders bevorzugt ist die Verwendung von Escherichia coli als Wirtsstamm für ein solches Expressionssystem.

Ein weiterer Gegenstand der Erfindung ist die Verwendung von Fnr - negativen Mikroorganismen als Wirtsstamm für ein erfindungsgemäßes Expressionssystem. Bevorzugterweise werden natürlicherweise Fnr - negative Mikroorganismen verwendet. Besonders bevorzugt ist die Verwendung von solchen Mikroorganismen als Wirtsstämme für ein erfindungsgemäßes Expressionssystem, bei denen das fnr - Gen durch dem Fachmann bekannte Methoden, inaktiviert worden ist. Ein Beispiel für einen solchen besonders bevorzugten Mikroorganismus ist E. coli RM101 (Sawers und Suppmann, (1992), J. Bacteriol. Vol 174, S. 3473-3478) und alle davon abgeleiteten Derivate.

Ein weiterer Gegenstand der Erfindung sind Fermentationsverfahren zur Produktion von Proteinen mittels Mikroorganismen, die dadurch gekennzeichnet sind, daß Mikroorganismen, die das erfindungsgemäße Expressionssystem enthalten, verwendet werden.

Soll bei einer erfindungsgemäßen Fermentation ein maximaler Induktionseffekt erreicht werden, wird ein ein erfindungsgemäßes Expressionssystem enthaltender Mikroorganismus bei pH - Werten > pH 7,0 unter aeroben Bedingungen ohne externe Zugabe von Acetat angezogen. Zu beliebigen Zeitpunkten ist es dann möglich, die Expression eines Gens für ein rekombinantes Protein, welches Teil der erfindungemäßen Expressionskassette ist, maximal zu induzieren, indem der Sauerstoffpartialdruck pO₂ auf Werte von 0 bis 5% begrenzt, der pH - Wert des Mediums auf pH 6,0 bis 6,5 reguliert und Acetat auf eine Endkonzentration von 40 mM bis 60mM zugegeben wird.

Durch geeignete Kombinationen verschiedenener Stellwerte dieser drei Stimuli ist es darüberhinaus einfach möglich, die Expression des Fusiongens in Zwischenstufen von sehr schwach bis sehr stark abgestuft zu induzieren. Die Möglichkeit zu einer genau abgestuften Induktion ist in der Regel sehr wünschenswert, denn dadurch ist es beispielsweise möglich den Expressionslevel so einzustellen, daß gerade die Menge des Zielproteins gebildet wird, bei der das Protein noch nicht als sog. Einschlußkörperchen ausfällt.

Eine Induktionsbedingung, die zu einer sehr schwachen Induktion führt, ist beispielsweise die Kombination pH-Wert > pH 6,5, externe Zugabe von Acetat (auf 40 mM) und die Gegenwart von Sauerstoff mit einem Partialdruck pO₂ von mehr als 10%.

Ein Vorteil des erfindungsgemäßen Expressionssystems ist es, daß es auch in Gegenwart von Sauerstoff (pO₂ > 5%) durch eine Reduktion des pH - Wertes des Anzuchtmediums auf pH 6,0 und die Zugabe von Acetat (40 bis 60 mM) eine starke Induktion der Expression entsprechender Fusionsgene ermöglicht.

Die folgenden Beispiele erläutern zusammen mit den Zeichnungen die Erfindung weiter. In den Zeichnungen stellt dar:
Fig. 1: Plasmidkarte von pBU1
Fig. 2: Plasmidkarte von pBTL142
Fig. 3: Nukleotidsequenz (Seq ID-No: 3) und Aminosäuresequenz (Seq ID-No: 4) der budA' - lacZ' - Übergangsstelle auf pBTL142 und pRBL2
Fig. 4: Plasmidkarte von pBAK1
Fig. 5: Plasmidkarte von pBAK14
Fig. 6: Plasmidkarte von pBAK16
Fig. 7 A: Größe und relative Orientierung der Gene des bud - Regulons im Genom von Klebsiella terrigena (DSM2687)
Fig. 7 B: Auf pBAK14 und pBAK16 enthaltenes DNS - Fragment
Fig. 7 C: Die Lage der zur PCR - Amplifikation von Klebsiella -DNS verwendeten Oligonukleotide (vgl. Beispiele 1 und 10)
Fig. 8: Plasmidkarte von pRBL2
Fig. 9: Konstruktion von pBUD100 (vgl. Beispiel 17)
Fig. 10: Nukleotidsequenz am Übergangsbereich bud-DNS - - CGTase - Strukturgen vor (pBUD100; Seq ID-No: 5) und nach (pBUD200; Seq ID-No: 6) gerichteter Mutagenese

### Allgemeines zur Konstruktion der in den Beispielen verwendeten Plasmide und den Expressionsstudien:

1. Alle anaeroben Anzuchten wurden nach Balch und Wolfe (1976), Appl. Environ. Microbiol. Vol. 32, p. 781-791 in Serumflaschen durchgeführt. Aerobe Anzuchten erfolgten in Erlenmeyerkolben unter starkem Schütteln (die Kolben wurden max. mit 1/10 des angegebenen Volumens gefüllt). Die Kulturen wurden bei 37 °C inkubiert.
2. Medium für aerobe Anzuchten: TGYEP (1% Trypton, 0,5% Hefeextrakt, 0,4% Glukose, 100mM K-Phosphat, pH-Werte mit 0,1 M Kaliumphosphat-Puffer auf 6,0 bis 8,0 eingestellt); Medium für aerobe Anzuchten: TGYEP (pH wie oben beschrieben eingestellt); Zusatz des Induktors Na-Acetat auf 40 mM aus einer 1 M Stammlösung.
   Zur Arbeit mit k-Phagen wurden die Medien nach Kleckner et al. ((1978), Genetics Vol 90, p. 427 - 450) eingesetzt.
3. Antibiotika wurden in folgenden Konzentrationen zugegeben: Ampicillin, 100 µg/ml; Chloramphenicol, 30 µg/ml; Kanamycin, 50 µg/ml; Tetracyclin, 20 µg/ml bzw. 15 µg/ml für chromosomal kodierte Resistenzen:
4. Präparation von chromosomaler DNS erfolgte nach der Methode von Ausubel et al. ((1987), Current protocols in molecular biology, Vol 1, Greene Publishing Associates and Wiley-Interscience, New York), Präparation von Plasmid-DNA nach der Methode von Holmes and Quigley ((1981), Anal. Biochem Vol 114, S. 193 - 197).
5. Transformation der verwendeten Stämme mit Plasmid-DNA erfolgte, falls nicht anders angegeben, nach Standardprozeduren (Maniatis et al. (1982), Molecular cloning, A Laboratory Manual, Cold Spring Harbor Laboratory, Cold Spring Harbor, N.Y., S. 249 - 255).
6. Bestimmung der β-Galaktosidase-Aktivität erfolgte nach Miller (1972) Experiments in molecular genetics, Cold Spring Harbor Laboratory, Cold Spring Harbor, N.Y.,S. 352 - 355. Die Enzymaktivitäten sind in Miller units angegeben.
7. Die Erzeugung einer lac⁻-Mutante von Klebsiella terrigena DSM2687 erfolgte durch ungerichtete UV-Mutagenese nach Miller (1972) Experiments in molecular genetics, Cold Spring Harbor Laboratory, Cold Spring Harbor, N.Y. S. 121 - 124. Die Bestrahlung erfolgte 80 Sekunden mit einer Intensität von 318 µW/cm². Die Stabilität der Mutation wurde durch Inkubation mit Nitrosoguanidin (Miller (1972) Experiments in molecular genetics, Cold Spring Harbor Laboratory, Cold Spring Harbor, N.Y., S. 125 - 129) auf Reversionen getestet. Nach Kontrolle der für Klebsiella typischen Stoffwechselreaktionen wurde für die weiteren Untersuchungen der Stamm KT14 ausgewählt.
8. Die Integration der budA-lacZ-Fusionen in das Chromosom von E. coli erfolgte gemäß der Methode von Simons et al. (1987), Gene Vol 53, S. 85 - 96. Ausgehend vom Stamm E. coli MC4100 (F-, araD139, D(argF-lac)U169, ptsF25, deoC1, relA1, flbB5301, rpsL150, 1-) (Casadaban und Cohen (1979), Proc Nat Acad Sci USA, Vol 76, S. 4530 - 4533) wurden folgende Transduktanden erhalten: BL142, und BL1. Ausgehend vom Stamm E. coli RM101 (fnr-) (Sawers und Suppmann (1992) J. of Bacteriology 174, 11 pp. 3474 - 3478) wurde durch Transduktion der Stamm BL12 erhalten.
9. Enzymatische in vitro - Reaktionen an DNS wurden, falls nicht anders angegeben, nach Maniatis et al. (1982), Molecular cloning, A Laboratory Manual, Cold Spring Harbor Laboratory, Cold Spring Harbor, N.Y. S. 98 - 148 durchgeführt.
   Ausgangsvektoren:
   1. pUC19 (Yanisch-Perron et al. (1985), Gene Vol 33, S.103 - 119)
   2. pBR322 (Bolivar et al. (1977), Gene Vol 2, S. 95-113)
   3. pRS552 (Simons et al. (1987), Gene Vol 53, S. 85-96)
   4. pJF118HE (Fürste et al. (1986), Gene Vol 48, S. 119-131)
   5. pCM100 (Binder et al. (1986), Gene Vol 47, S. 269 - 277)
   Phagen:
   kRS45 (Simons et al. (1987), Gene Vol 53, S. 85 - 96)
10. Synthetische Oligonukleotide wurden von der Firma Toplab (Martinsried, Germany) bezogen. Die Nukleotidsequenzen der in den Beispielen verwendeten Oligonukleotide sind in Tab. 1 zusammengefaßt: 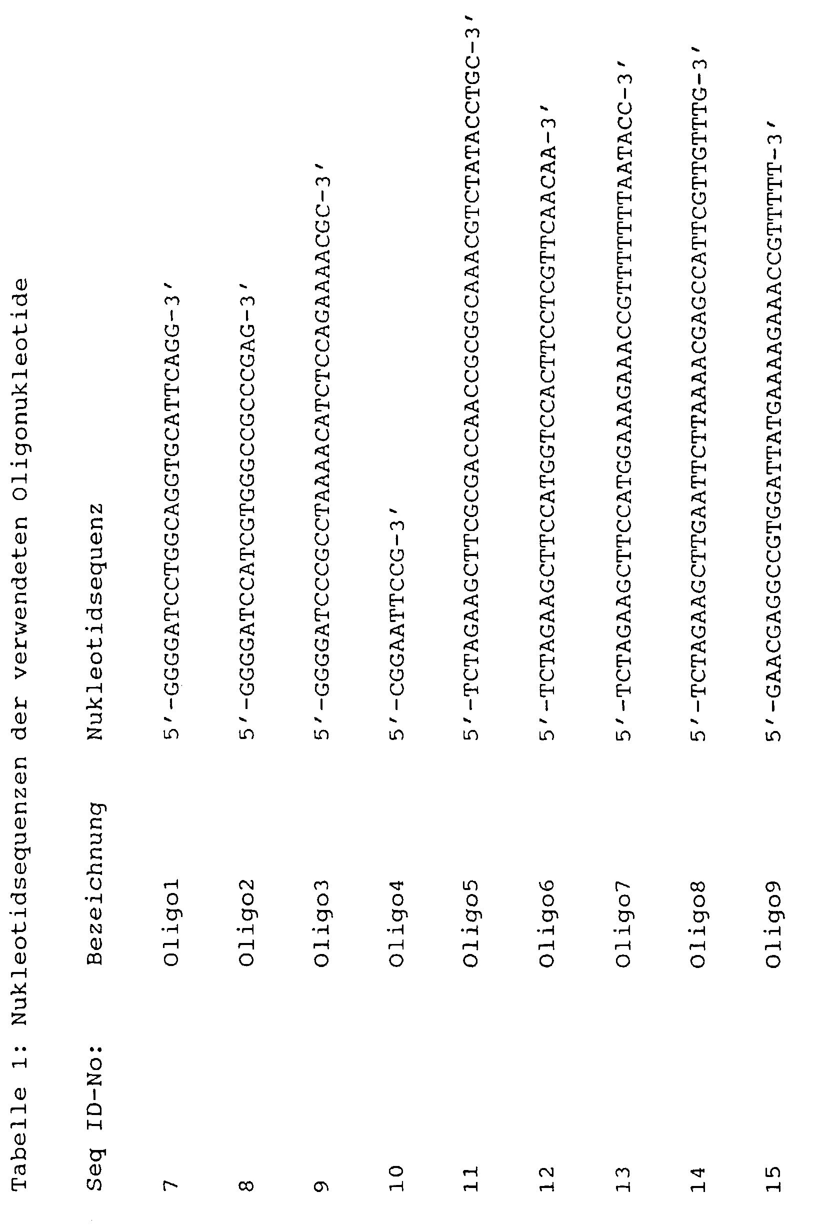
11. Enzyme zur Restriktion, Modifikation und Sequenzanalyse von DNA wurden von Boehringer Mannheim GmbH (Mannheim), Pharmacia (Freiburg), New England Biolabs (Schwalbach) und Perkin Elmer Cetus (Langen) bezogen.

Radioaktive Substanzen stammten von Amersham Buchler (Braunschweig) oder NEN Du Pont (Dreieich).

Feinchemikalien waren von den Firmen Merck (Darmstadt), Sigma (München), Serva (Heidelberg), Fluka (Neu-Ulm) und Biomol (München).

### Beispiel 1: Translationelle Kopplung des budA - Promotorfragments mit dem lacZ-Gen

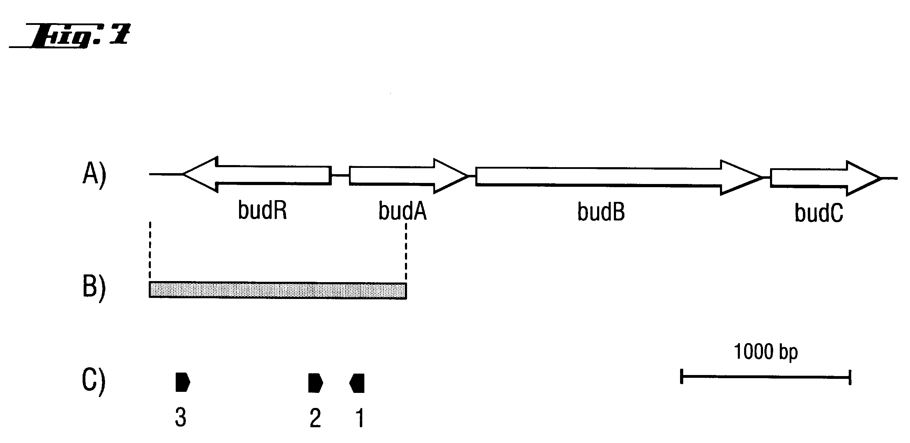

Mit Hilfe der Oligonukleotide Oligol und Oligo2 (Tabelle 1), deren Nukleotidsequenz aus der von Blomquist et al. (J. Bact. (1993) Vol 175(5), S. 1392 - 1404) veröffentlichten Sequenz des bud - Operons aus Klebsiella terrigena abgeleitet wurde, wurde ein 223 bp großes Fragment aus chromosomaler DNA aus Klebsiella terrigena (DSM2687) durch symmetrische PCR amplifiziert (Annealing: 30 s bei 58 °C; Kettenverlängerung: 60 s bei 72 °C; Strangtrennung: 30 s bei 94 °C; 25 Zyklen). Das erhaltene Fragment erstreckt sich über die Positionen 247 bis 456 der Nukleotidsequenz Seq ID-No:2 und enthält die Sequenzinformation für die 10 N-terminalen Aminosäurereste von BudA (vgl. Fig. 7 A), sowie 178 stromaufwärts vom budA - Startcodon gelegene Nukleotide, welche den funktionell aktiven Promotor des bud - Operons enthalten.

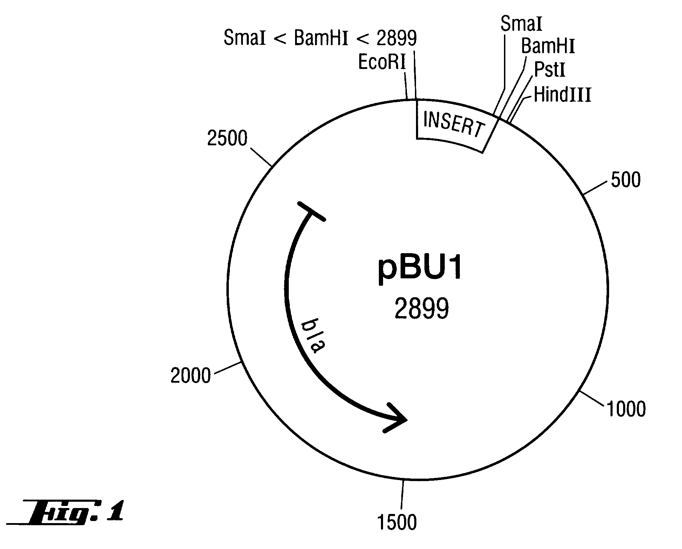

Unter Verwendung der durch die PCR - Startnukleotide Oligo 1 und Oligo 2 eingeführten BamHI - Restriktionserkennungsstellen wurde nach Spaltung des PCR - Produkts mit BamHI ein 219 bp großes DNS - Fragment isoliert und in den mit BamHI geschnittenen Vektor pUC19 kloniert. Die korrekte Nukleotidsequenz, sowie die Orientierung des Inserts wurde durch doppelsträngige Sequenzanalyse (Sanger et al. (1977) Proc.Natl.Acad.Sci.USA 74(12), pp.5463 - 5467) überprüft. Das hieraus resultierende Konstrukt wurde pBU1 (Fig. 1) genannt.

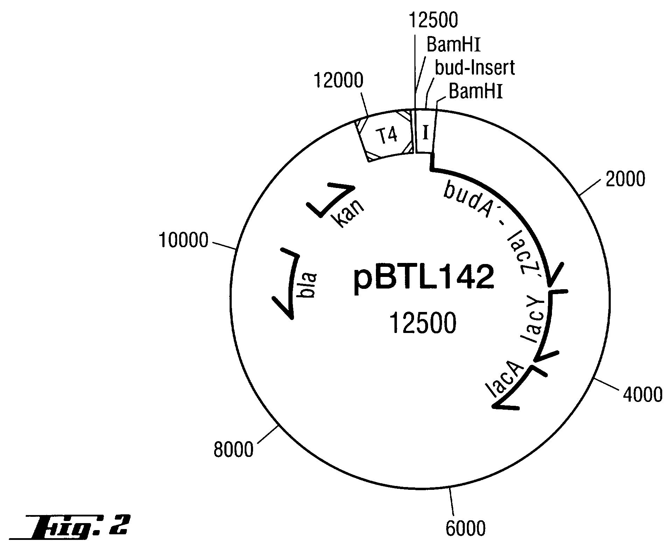

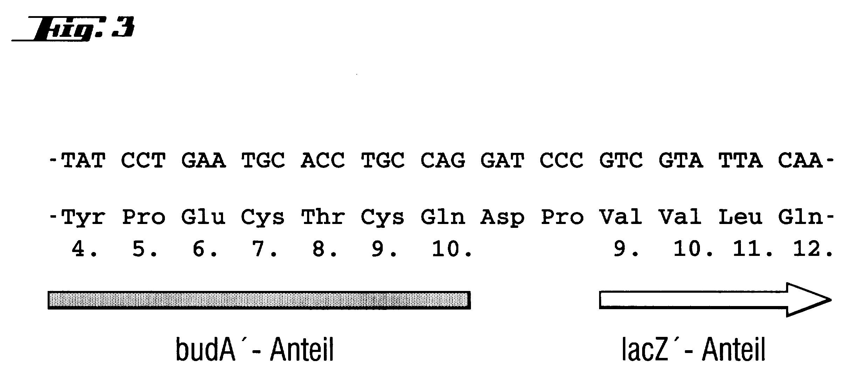

Zur Kombination des bud - Promotors auf pBU1 mit dem lacZ - Gen als Reportergen für eine Promotoraktivität wurde das BamHI-Fragment aus pBU1 in die BamHI-Schnittstelle des Promotortest - Vektors pRS552 umkloniert. Das erhaltene Konstrukt, Plasmid pBTL142 (Fig. 2), kodiert für ein Fusionsprotein, bestehend aus den 10 N-terminalen Aminosäure-Resten von BudA und daran über einen Linker aus 2 Aminosäureresten anschließend LacZ ab dem 9. Aminosäurerest (Fig. 3, Seq ID-No: 3 und 4), dessen Transkription nur von den in den 158 bp vor budA enthaltenen Regulationselementen gesteuert werden kann.

### Beispiel 2: Expression von β-Galaktosidase-Protein durch das bud-Promotorfragment auf pBTL142 in Klebsiella terrigena KT14

Zur funktionellen Untersuchung des bud-Promotors auf pBTL142 in Klebsiella terrigena (homologes System) wurde Klebsiella terrigena KT14 (lacZ-) durch Elektroporation (Fiedler und Wirth (1988) Analytical Biochemistry 170, pp.38 - 44) mit pBTL142 transformiert. Für Expressionsstudien wurde ein dabei erhaltener Transformand (Klebsiella terrigena KT14/pBTL142) unter den in Tab. 2 dargestellten Bedingungen angezogen. Die dabei gebildeten β-Galaktosidase - Aktivitäten (Tab. 2), welche nach Miller aus Zell - Lysaten bestimmt wurden, sind ein direktes Maß für die Aktivität des bud-Promotors unter den jeweiligen Bedingungen.

**Tabelle 2**

| Expression von budA'-lacZ' in Klebsiella terrigena KT14/pBTL142 | | | | |
|---|---|---|---|---|
| pH | +O₂ | +O₂ + Acetat | -O₂ | -O₂ + Acetat |
| 6,0 | 668 | 5688 | 2300 | 11515 |
| 6,5 | 800 | 2272 | 1057 | 4326 |
| 7,0 | 833 | 1280 | 760 | 1533 |
| 7,5 | 884 | 1123 | 784 | 754 |
| 8,0 | 875 | 1003 | 741 | 743 |

### Beispiel 3: Expression von β-Galaktosidase - Protein in Klebsiella terrigena KT14/pBTL142 bei Fermentation unter induzierenden und nicht induzierenden Bedingungen

Fermentertyp: Biostat ED (B. Braun Biotech, Melsungen, Deutschland).

Füllvolumen: 7 l

Medium: KH₂PO₄: 1,5 g/l; (NH₄)₂SO₄: 5,0 g/l; NaCl: 0,5 g/l; FeSO₄ x 2H₂O: 0,075 g/l; Na₃Citrat x 2H₂O: 1,0 g/l; Trypton (Oxoid): 5 g/l; Hefeextrakt (Oxoid): 2,5 g/l; Glukose: 12 g/l; Kanamycin: 50 mg/l

pH des Mediums: Konstant 6,0 (Korrektur mit 6N NH₄OH, bzw. 4N H₃PO₄)

Temperatur: 30°C

Belüftung: Die Anzuchten wurden über den gesamten Fermentationsverlauf konstant mit 4 l Luft pro Minute belüftet. Bis zum Erreichen einer optischen Dichte von OD₆₀₀ = 20 wurde ein konstanter Sauerstoff - Partialdruck von pO₂ = 40% durch Rühren mit Geschwindigkeiten zwischen 450 bis 900 rpm eingehalten. Bei Erreichen von OD₆₀₀ = 20 wurde innerhalb einer Stunde der pO₂ durch Reduktion der Rührgeschwindigkeit auf pO₂ = 0% eingestellt und für 48 h gehalten.

Induktion: Bei Erreichen von pO₂ = 0% wurde den zu induzierenden Fermentern Acetat zu einer Endkonzentration von 40 mM zugesetzt.

Die β-Galaktosidase - Aktivitäten wurden 24h und 48h nach Induktion ermittelt

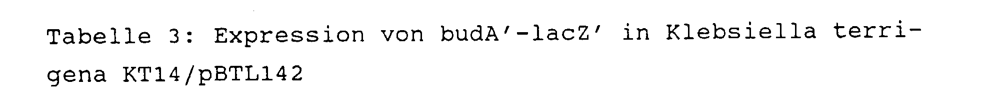

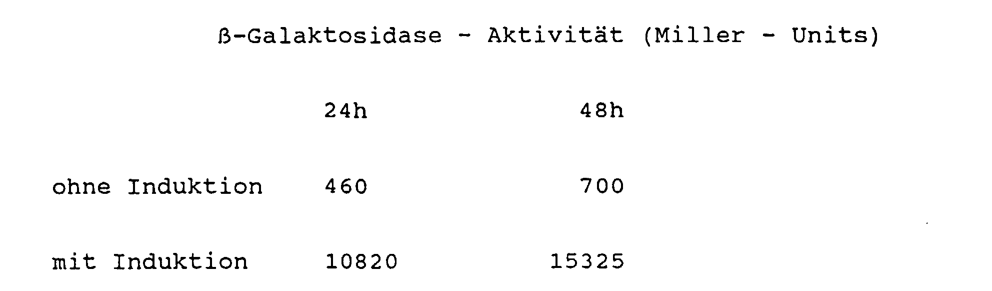

### Beispiel 4: Expression von β-Galaktosidase-Protein durch das budA-Promotorfragment auf pBTL142 in E. coli FM420

Nach Transformation von E. coli FM420 mit pBTL142 wurden bei anschließenden Expressionsstudien die in Tabelle 4 dargestellten Werte ermittelt.

**Tabelle 4**

| Expression von budA'-lacZ' in E. coli FM420/pBTL142 | | | |
|---|---|---|---|
| pH | +O₂ | -O₂ | -O₂ + Acetat |
| 6,0 | 424 | 408 | 737 |
| 7,0 | 613 | 466 | 722 |
| 8,0 | 655 | 607 | 794 |

Im heterologen System ist keine signifikante Induktion des bud - Promotors erkennbar. E. coli besitzt nicht die zur vollen Expression und Regulation erforderlichen Transkriptionsfaktoren, d. h. die auf pBTL142 vorhandenen, cis - wirksamen Faktoren sind alleine nicht ausreichend, um das in Klebsiella terrigena (Bsp. 2) beobachtete Expressionsverhalten in E. coli zu vermitteln.

### Beispiel 5: Integration der budA '- 'lacZ-Translationsfusion auf Plasmid pBTL142 in das Genom von E. coli MC4100

Die Integration der budA '- 'lacZ-Fusion in das Chromosom erfolgte gemäß der Methode von Simons et al. ((1987), Gene Vol 53, S. 85 - 96). Durch Transformation von pBTL142 in E. coli MC4100 und anschließender Infektion mit kRS45 wurde die Translationsfusion in das Chromosom von E. coli MC4100 integriert. Erfolgreiche Integration wurde anhand der transduzierten Kanamycin-Resistenz überprüft. Nach UV-Bestrahlung erhaltene Lysate von kBTL142 (gereinigte Phagenlinie) wurden zur erneuten Transduktion von E. coli MC4100 benutzt und der so konstruierte Stamm als E. coli BL142 bezeichnet.

### Beispiel 6: Expression von budA'-lacZ'- Protein durch das chromosomal integrierte Gen in E.coli BL142

Alle Anzuchten wurden bei pH 6,5 durchgeführt.

**Tabelle 5**

| Expression von budA'-lacZ' in E. coli BL142 | |
|---|---|
| Wachstumsbedingungen | β-Galaktosidase-Aktivität |
| +O₂ | 35 |
| -O₂ | 40 |
| -O₂ + Acetat | 70 |

Unter den angegebenen Wachstumsbedingungen ist eine geringe Grundexpression, aber keine signifikante Induktion durch Anaerobiose und Acetat-Zugabe zu erkennen.

### Beispiel 7: Klonierung eines für ein den budA-Promotor in E. coli aktivierendes Protein kodierenden Gens aus Klebsiella terrigena

Chromosomale DNA aus Klebsiella terrigena DSM 2687 wurde isoliert und mit Sau3A partiell verdaut (0,02 units/µg DNA, 20 min bei 37 °C). Fragmente zwischen 3 und 10 kb wurden nach elektrophoretischer Auftrennung isoliert und in den mit BamHI linearisierten Vektor pBR322 ligiert. Der nach Transformation von E. coli JM109 und anschließender Präparation erhaltene Plasmid-Pool wurde als Genbank von Klebsiella terrigena benutzt.

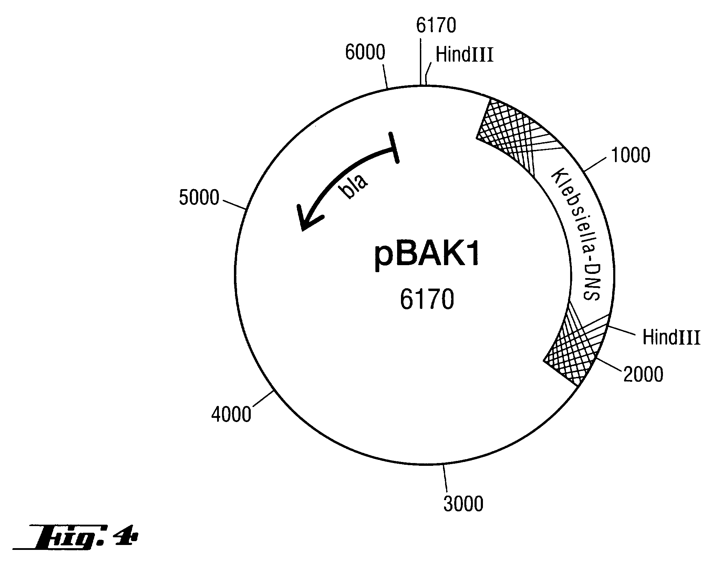

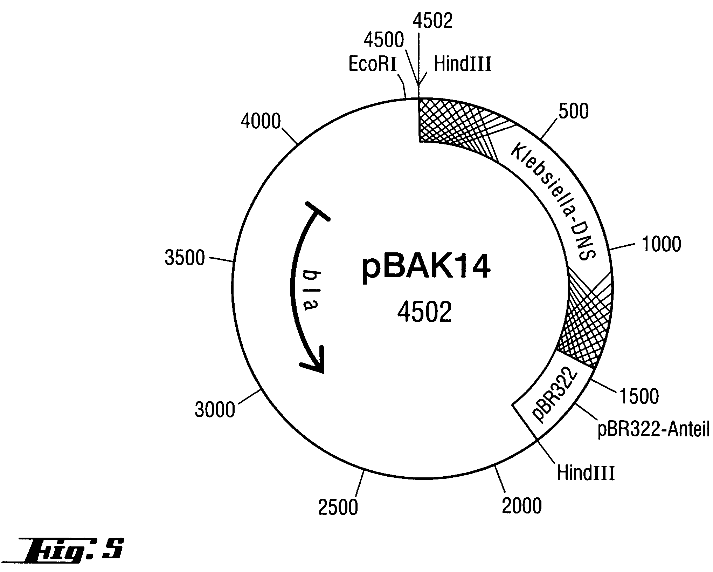

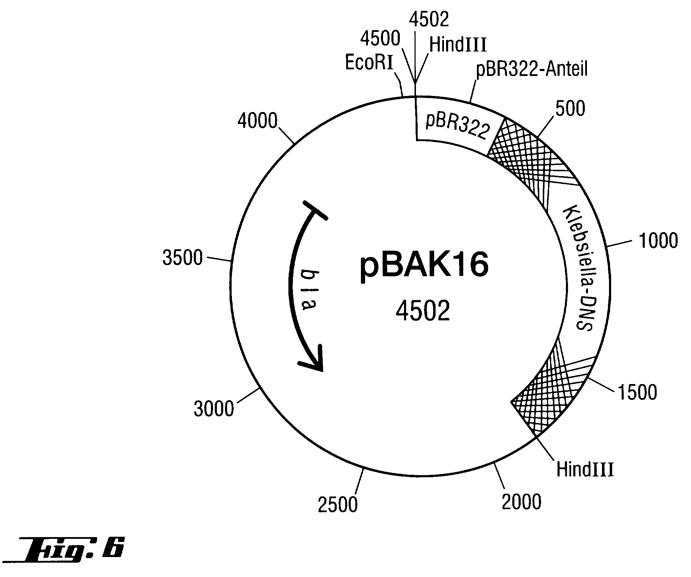

Zur Identifikation von Plasmiden, die für einen den bud - Promotor in Gegenwart von Acetat aktivierenden, in trans wirksamen Faktor kodieren wurde E. coli BL142 mittels Elektroporation mit der Genbank aus Klebsiella terrigena transformiert (Fiedler und Wirth (1988) Analytical Biochemistry 170, pp. 38 - 44). Die Transformationsansätze wurden auf sogenannte Indikatorplatten ( Kaliumphosphatgepufferter TGYEP-Agar (pH 6,5) mit 0,4 % Glukose, 40 mM Acetat, 1 mM X-Gal und Ampicillin (100 µg/ml)) ausgebracht und bei 37°C inkubiert. E. coli BL142 bildet auf diesen Indikatorplatten aufgrund seiner sehr schwachen β - Galaktosidase - Aktivität (Tab. 5) hellblaue Kolonien. Ein Klon bildete dagegen nach der Transformation eine tiefdunkelblaue Kolonie. Er enthielt das Plasmid pBAK1 (Abb. 4), welches ein ca. 1,8 kb großes Sau3A - Fragment aus Klebsiella terrigena trägt. Für die weiteren Analysen wurde ein 1,8 kb großes HindIII - Fragment aus pBAK1 (vgl. Abb. 4), welches ein 350 bp großes Fragment des Vektors pBR322 und ein 1,45 kb großes Fragment aus Klebsiella terrigena enthält, isoliert; die überhängenden Enden des Fragments wurden mit Klenow - Polymerase aufgefüllt. Anschließend wurde das Fragment in beiden Orientierungen in das mit SmaI linearisierte Plasmid pUC19 ligiert. Die daraus entstandenen Plasmide, die auf Indikatorplatten ebenfalls eine Blaufärbung von Kolonien von E. coli BL142 vermitteln, wurden als pBAK14 und pBAK16 (Abb. 5 und 6) bezeichnet.

### Beispiel 8: Sequenzanalyse des Gens für das den budA-Promotor aktivierende Regulatorprotein auf pBAK14 und pBAK 16

Zur Sequenzanalyse des Inserts auf pBAK14 und pBAK16 nach Sanger et al. ((1977), Proc Natl Acad Sci USA Vol 74, S. 5463 - 5467) wurden die Inserts jeweils mittels Exonuklease III verkürzt. Als Vektorschutz diente die Restriktion mit SacI, Exonuklease-Angriff erfolgte an einer Asp718-Schnittstelle der multiplen Klonierungs-site in pUC19 (Henikoff (1984), Gene Vol 28, S. 351 - 359). Die Sequenzreaktionen mit T7-DNA-Polymerase (Pharmacia, Freiburg) wurden parallel mit dGTP und dITP durchgeführt, um starke Kompressionen zu vermeiden. Ein für pUC-Vektoren spezifisches, käuflich erhältliches Universal-Sequenzierstartoligonukleotid wurde verwendet. Für das DNS -Insert aus Klebsiella terrigena auf pBAK14 und pBAK16 wurde die folgende Nukleotidsequenz (Seq ID-No:2) ermittelt:

Aus der ermittelten Nukleotidsequenz (Seq ID-No: 2) konnte ein offener Leserahmen (Nukleotid 385 bis 1254) abgeleitet werden, der für ein Protein (Seq ID-No: 1), bestehend aus 290 Aminosäuren, kodiert. Die aus diesem offenen Leserahmen abgeleitete Aminosäuresequenz lautete wie folgt:

Aufgrund seiner die Aktivität des bud - Promotors regulierenden Aktivität wurde dieses Protein als BudR (Bud -Regulator) bezeichnet. Die Transkriptionsrichtung von budR ist gegenläufig zu der des bud - Operons, allerdings befindet sich zwischen bud - Operon und budR nur eine intergene Region von lediglich 106 bp (Nukleotid 279 bis 384 in Seq ID-No: 2). bud - Operon und budR bilden damit ein divergent orientiertes Regulon für die Synthese der an der Bildung von 2,3 - Butandiol beteiligten Enzyme (Fig. 7).

### Beispiel 9: Expression von β-Galaktosidase-Protein durch die chromosomal vorliegende budA '-'lacZ-Translationsfusion in E. coli BL142/pBAK1

Die Induzierbarkeit der chromosomal codierten budA '-'lacZ-Fusion durch Plasmide der Genbank, die einen lac+ Phänotyp (Blaufärbung) hervorrufen, soll an folgendem Beispiel demonstriert werden. Alle Anzuchten erfolgten bei pH 6,5.

**Tabelle 6**

| Expression von budA'-lacZ' in E. coli BL142/pBAK1 | |
|---|---|
| Wachstumsbedingungen | β-Galaktosidase-Aktivität |
| +O₂ | 1832 |
| -O₂ | 1147 |
| -O₂ + Acetat | 5802 |

### Beispiel 10: Konstruktion der budA '-'lacZ-Fusion zusammen mit der vollständigen Sequenz von budR und der intergenen Region zwischen budR und budA

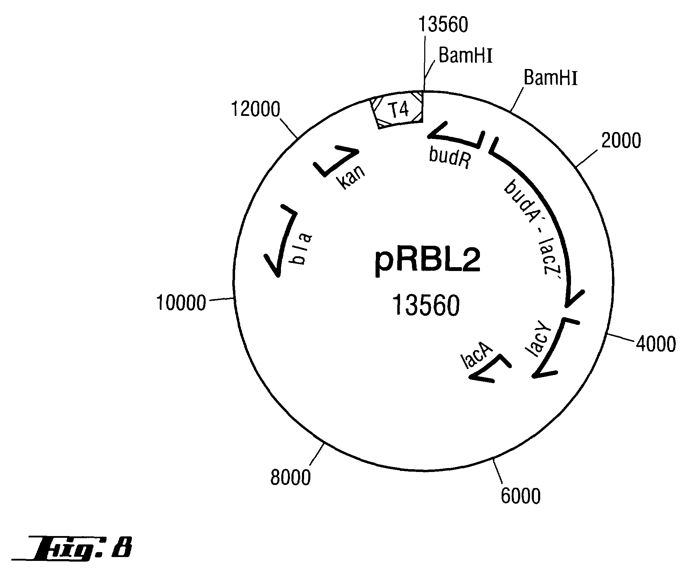

Mit Hilfe der Oligonukleotide Oligol und Oligo3 (Tab. 1) wurde über symmetrische PCR aus pBAK16 (pUC19-Derivat) ein 1027 bp großes Fragment amplifiziert (Abb. 7). Unter Verwendung der durch die PCR - Startnukleotide Oligo 1 und Oligo3 eingeführten BamHI - Restriktionserkennungsstellen wurde nach Spaltung des PCR - Produkts mit BamHI ein 1023 bp großes DNS - Fragment isoliert und in den mit BamHI geschnittenen Vektor pRS552 kloniert. Die korrekte Orientierung des Inserts wurde durch Sequenzanalyse Sanger et al. ((1977), Proc Natl Acad Sci USA Vol 74, p. 5463 - 5467) überprüft. Das hieraus resultierende Konstrukt wurde pRBL2 (Fig. 8) genannt.

Plasmid pRBL2 enthält das vollständige Gen für BudR, die komplette intergene Region zwischen budR und budA sowie das Gen, kodierend für ein Fusionsprotein, bestehend aus den 10 N-terminalen AS - Resten von BudA und daran über einen Linker aus 2 Aminosäureresten anschließend LacZ ab dem 9. Aminosäurerest, das auch auf pBTL142 enthalten ist (Fig. 3).

### Beispiel 11: Integration von budR in das Genom von E. coli MC4100

Die Integration der budA '-'lacZ Fusion mit der zusätzlichen Sequenz von budR auf Plasmid pRBL2 in das Chromosom von E. coli MC4100 erfolgte gemäß der Methode von Simons et al. (1987), Gene Vol 53, p. 85 - 96. Durch Transformation von pRBL2 in E. coli MC4100 und anschließender Infektion mit λRS45 von E. coli MC4100/pRBL2 wurde die Fusion in das Chromosom von E. coli MC4100 integriert. Erfolgreiche Integration wurde anhand der transduzierten Kanamycin-Resistenz überprüft. Die nach UV-Bestrahlung erhaltenen Lysate von RBL2 (gereinigte Phagenlinien) wurden zur erneuten Transduktion von E. coli MC4100 benutzt und der so konstruierte Stamm E. coli BL2 genannt.

### Beispiel 12: Expression von β-Galaktosidase-Protein durch die chromosomal vorliegende budA '-'lacZ-Fusion in E. coli BL2

Die durch die einfache Kopie von budR auf die Expression des budA'-lacZ'-Fusionsgens im Chromosom von E. coli erzielte Regulation ist in Tabelle 7 dargestellt. Alle Anzuchten erfolgten im TGYEP-Medium bei pH 6,5.

**Tabelle 7**

| Expression von budA'-lacZ' in E. coli BL2 | |
|---|---|
| Wachstumsbedingungen | β-Galaktosidase-Aktivität |
| -O₂ | 99 |
| -O₂ + Acetat | 1130 |

### Beispiel 13: Expression von β-Galaktosidase-Protein in E. coli BL2/pBTL142

Die Induktion des plasmidcodierten budA'-lacZ' - Fusiongens durch das chromosomal codierte BudR ist in Tabelle 8 dargestellt.

**Tabelle 8**

| Expression von budA'-lacZ' in E. coli BL2/pBTL142 | | | | |
|---|---|---|---|---|
| pH | +O₂ | +O₂ + Acetat | -O₂ | -O₂ + Acetat |
| 6,0 | 1435 | 10114 | 1520 | 14413 |
| 6,5 | 1410 | 6907 | 928 | 5381 |
| 7,0 | 1403 | 2257 | 980 | 1968 |
| 7,5 | 1281 | 1440 | 960 | 1339 |
| 8,0 | 1265 | 1665 | 1135 | 1243 |

### Beispiel 14: Stufenweise Deletion des 5'-Bereichs der budA-Promotorregion auf Plasmid pBTL142

Um den minimalen Nukleotidbereich des bud-Promotors zu ermitteln der noch eine durch budR aktivierbare Promotoraktivität besitzt, wurde der auf Plasmid pBTL142 vorhandene Promotor von seiner 5' - Seite aus stufenweise verkürzt. Dazu wurde pBU1 durch Restriktion mit EcoRI am 5'-Ende des Inserts linearisiert und mit Bal31-Exonuklease (Boehringer Mannheim) bei 30 °C inkubiert (0,3 Units/µg DNA) (Schaffner et al., 1976). Die 5'-überhängenden DNA-Enden wurden durch das Klenow-Fragment der DNA-Polymerase I aufgefüllt und mit EcoRI-Linkern (Oligo4 (Tab. 1)) versehen. Nach Restriktion mit EcoRI und BamHI wurden die Fragmente elektrophoretisch aufgetrennt, Fragmente der geeigneten Länge wurden eluiert und in den mit EcoRI und BamHI vorbehandelten Vektor pRS552 eingeführt. Die 5'-Enden der Inserts in den Deletionskonstrukten wurden durch Sequenzanalyse bestimmt. Ausgewählte Klone (pBTL6 bis pBTL124) sind in Tabelle 9 aufgelistet.

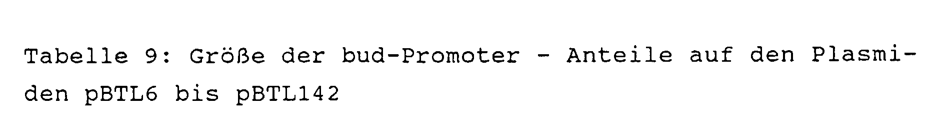

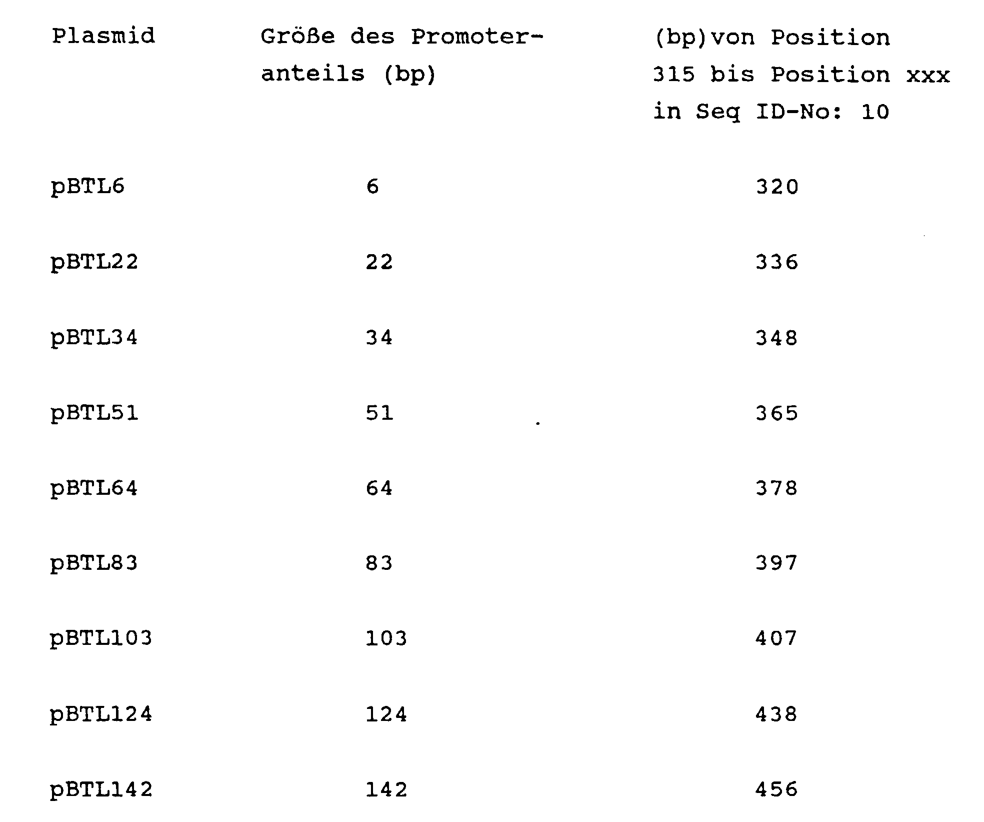

### Beispiel 15: Expression von β-Galaktosidase-Protein durch die verkürzten budA-Promotorfragmente auf den Plasmiden pBTL6 bis pBTL142

Expressionsstudien mit den Verkürzungsklonen wurden in Klebsiella terrigena KT14 als Wirtsstamm durchgeführt. Alle Anzuchten erfolgten bei pH 6,5:

**Tabelle 10**

| Expression von budA'-lacZ' in Klebsiella terrigena KT14/pBTL6 - pBTL142 | | | | |
|---|---|---|---|---|
| Plasmid | +O₂ | +O₂ + Acetat | -O₂ | -O₂ + Acetat |
| pBTL6 | 30 | 49 | 18 | 30 |
| pBTL22 | 52 | 136 | 41 | 120 |
| pBTL34 | 34 | 70 | 24 | 84 |
| pBTL51 | 264 | 645 | 205 | 523 |
| pBTL64 | 252 | 677 | 214 | 624 |
| pBTL83 | 398 | 2575 | 967 | 5563 |
| pBTL103 | 211 | 1675 | 752 | 4703 |
| pBTL124 | 238 | 1849 | 828 | 4444 |
| pBTL142 | 449 | 2040 | 767 | 3145 |

### Beispiel 16: Expression von β-Galaktosidase-Protein in E. coli BL12 und E. coli BL12/pUFR1

Um den Einfluß des E. coli eigenen Induktors des anaeroben Stoffwechsels, Fnr, auf die BudR - abhängige Aktivierung des bud-Promotors zu untersuchen, wurde budR sowie die budA'-lacZ' - Translationsfusion auf Plasmid pRBL2 (Fig. 8) über Transduktion mit λRS45 analog der Vorgehensweise aus Bsp. 11 in das Chromosom des fnr - negativen E. coli RM101 integriert. Der dabei erhaltene Stamm wurde als E. coli BL12 bezeichnet. Um mögliche Fnr - Effekte zu untersuchen, wurde E. coli BL12 zusätzlich mit dem ein funktionelles fnr - Gen tragenden Plasmid pUFR1 (Sawers und Suppmann (1992) J. of Bacteriology 174, 11 pp. 3474 - 3478) transformiert.

**Tabelle 11**

| Expression von budA'-lacZ' in E. coli BL12 und BL12/pUFR1 | | |
|---|---|---|
| Stamm/Plasmid | Wachstumsbedingungen | β-Galaktosidase-Aktivität |
| BL12 | -O₂ | 371 |
| BL12 | -O₂ + Acetat | 3700 |
| BL12/pUFR1 | -O₂ | 115 |
| BL12/pUFR1 | -O₂ + Acetat | 509 |

### Beispiel 17: Funktionelle Kopplung des bud - Regulatorsystems mit dem alpha-CGTase - Strukturgen

Für die Konstruktion des CGTase - Expressionsplasmides pBUD200 wurden die in Tabelle 1 dargestellten Oligonukleotide Oligo5 bis Oligo9 verwendet.

### A) Konstruktion von pBUD100

Ein das vollständige budR - Gen und den budA - Promotor enthaltendes DNS - Fragment (Nukleotide 281 bis 1300 in SEQ ID-No: 2) wurde mit Hilfe der Oligonukleotide Oligo5 und Oligo6 (Tab. 1) unter Verwendung von Plasmid pBAK16 als Vorlagen - DNS mittels PCR amplifiziert und mit den Restriktionsendonukleasen NruI und NcoI gespalten.

Für die Amplifikation des Strukturgens der alpha-CGTase aus Klebsiella oxytoca M5a1 wurden die Oligonukleotide Oligo7 und Oligo8 (Tab. 1) als Startoligonukleotide, sowie Plasmid pCM100 (Binder et al. (1986), Gene Vol 47, p. 269 - 277) als Vorlagen - DNS verwendet. Das amplifizierte DNS - Fragment wurde mit den Restriktionsendonukleasen NcoI und EcoRI gespalten.

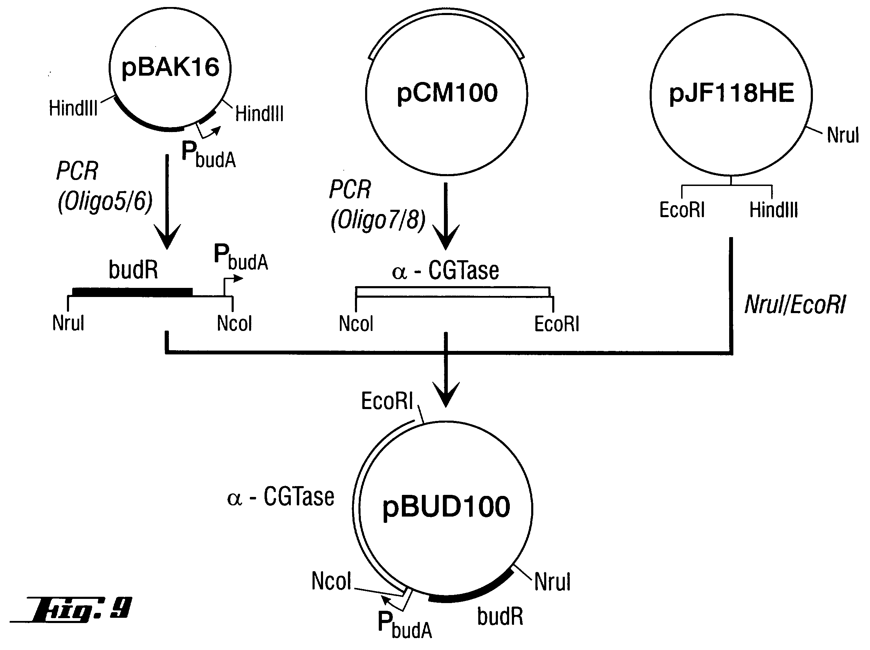

Die beiden PCR - Fragmente wurden zusammen in den mit NruI und EcoRI gespaltenen Vektor pJF118HE ligiert (Fig. 9). Das dabei entstandene Plasmid wird als pBUD100 bezeichnet.

### B) Konstruktion von pBUD200 durch gerichtete Mutagenese

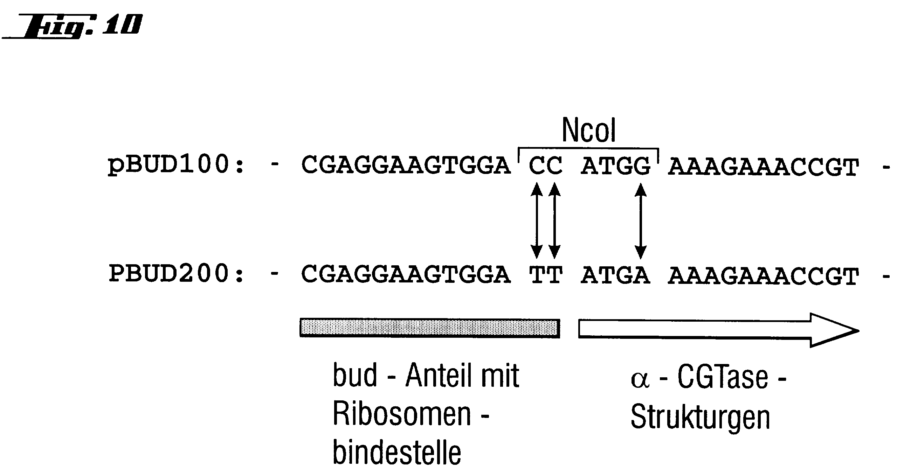

Um eine effektive Translation des alpha-CGTase - Gens auf pBUD100 zu ermöglichen, wurden drei Punktmutationen, welche bei der Konstruktion von pBUD100 erzeugt worden sind, um über eine NcoI - Erkennungsstelle den budA - Promotor und das alpha-CGTase - Strukturgen verknüpfen zu können, revertiert (Fig. 10).

Durch gerichtete Mutagenese nach Deng & Nickoloff (1992), Anal. Biochem. Vol. 200, p. 81 ff wurde unter Verwendung von Oligo9 (Tab. 1) als Mutageneseoligo die DNS -Sequenz im Bereich des Übergangs an die Originalsequenz von budA bzw. alpha-CGTase angepaßt. Die Positionen der mutagenisierten Basen sind Fig. 10 (Seq ID.No: 5 und 6) zu entnehmen. Das mutagenisierte Plasmid wird als pBUD200 bezeichnet.

### Beispiel 18: Enzymtest zur Bestimmung der alpha-CGTase - Aktivität

Die Bestimmung der Aktivität von CGTasen erfolgte nach der von Candussio et al. in Eur. J. Biochem. (1990) 191, S. 177 - 185 beschriebenen Methode.

Jeweils 2 Einheiten pro Gramm Stärke einer zu testenden CGTase wurden mit einer 5 % - igen (w/v) Lösung einer löslichen Stärke (Merck, Darmstadt) in einem Puffer bestehend aus 20 mM Tris/HCl pH 7,2 und 5 mM CaCl₂ für eine definierte Zeit bei 45°C inkubiert. Anschließend wurde die Reaktion durch die Zugabe von 1,5 Volumenteilen Methanol beendet. Nicht umgesetzte Reststärke wurde durch eine einstündige Inkubation bei 4°C gefällt und durch Zentrifugation (10 min, 12000 x g ) abgetrennt. Die entstandenen Produkte wurden über HPLC an einer Nukleosil 10-NH2-Säule (Macherey & Nagel, Düren) bestimmt, wobei definierte Cyclodextrine (Wacker-Chemie, München) als Standard dienten.

### Beispiel 19: Expression von alpha-CGTase durch das bud - Regulator/Promotor - System auf Plasmid pBUD200 in E. coli WCM105

Zur durch Sauerstoff, pH - Wert und/oder Acetat steuerbaren Produktion von alpha-CGTase wurde das in Bsp. 16 beschriebene Expressionsplasmid pBUD200 in einen E. coli - Sekretionsstamm transformiert. Als E. coli - Sekretionsstamm wurde E. coli WCM105 verwendet. Dieser Stamm wurde wie in EP 338410 beschrieben aus E. coli DS410 hergestellt.

Zum Nachweis der steuerbaren alpha-CGTase - Produktion wurde E. coli WCM105/pBUD200 bei 37°C in Kaliumphosphat - gepuffertem Vollmedium (TGYEP mit pH 6,5 bzw. pH 8,0; Begg et al. (1977), FEMS Microbiol. Lett. Vol 2, p. 47 - 50) unter Zusatz von 0,4 % Glukose (w/v) und ggf. 40 mM Natriumacetat angezogen. Anaerobe Kultivierung erfolgte in Serumflaschen nach der Technik von Balch und Wolfe (1976). Bei einer OD600 zwischen 0,8 und 1,0 wurden die Zellen durch Zentrifugation bei 5000 x g abgetrennt. Der zellfreie Kulturüberstand wurde, wie in Bsp. 15 beschrieben, zur Bestimmung der darin enthaltenen alpha-CGTase - Aktivität verwendet. Die Resultate sind in Tab.12 zusammengefaßt:

**Tabelle 12:**

| alpha-CGTase - Aktivität im Überstand von E. coli WCM105/pBUD200 | | | |
|---|---|---|---|
| (Bei den dargestellten Werten handelt es sich jeweils um den Mittelwert aus zwei parallelen Versuchsansätzen) | | | |
| pO₂ | pH | Acetat (mM) | CGTase-Aktivität (mU/10⁻¹·ml⁻¹) |
| aerob | 6,5 | 0 | 2.5 |
| anaerob | 6,5 | 0 | 30,0 |
| anaerob | 6,5 | 40 | 64,0 |
| aerob | 8,0 | 0 | < 1,0 |
| anaerob | 8,0 | 0 | 24,0 |
| anaerob | 8,0 | 40 | 46,5 |

## Patentansprüche

1. Durch Acetat, pH-Wert und Sauerstoff steuerbares Expressionssystem, umfassend ein in trans wirkendes Regulatorprotein und einen von diesem Protein aktivierbaren Promotor, dadurch gekennzeichnet, daß das Regulatorprotein eine Aminosäuresequenz, die zu mindestens 75% homolog zu der Aminosäuresequenz Seq. ID-No: 1 ist, umfaßt und der Promotor eine DNS-Sequenz umfaßt, die zu mindestens 95% homolog zu den Basen 315 bis 397 der DNS-Sequenz Seq. ID No: 2 ist.

2. Expressionssystem nach Anspruch 1, dadurch gekennzeichnet, daß es beliebige Strukturgene unter Kontrolle des Expressionssystems bei einem Sauerstoffpartialdruck pO₂ von 0-5%, einem pH-Wert von 6,0-6,5 und in Gegenwart von Acetat in einer Konzentration von 40-60 mM maximal exprimiert

3. Regulatorprotein, dadurch gekennzeichnet, daß es bei Sauerstofflimitierung und Anwesenheit von Acetat und einem pH-Wert des Anzuchtmediums von pH 6,0 bis pH 6,5 eine optimale Aktivierung des bud - Promotors aus Klebsiella terrigena (DSM2687) bewirkt.

4. Regulatorprotein nach Anspruch 3, dadurch gekennzeichnet, daß es eine Aminosäuresequenz umfaßt, die zu mindestens 75% homolog zu der Aminosäuresequenz Seq. ID-No.1 ist.

5. Gen kodierend für ein Protein gemäß Anspruch 3 oder 4.

6. Expressionskassette, dadurch gekennzeichnet, daß ein Promotor, der eine DNS-Sequenz umfaßt, die zu mindestens 95% homolog zu den Basen 315 bis 397 der DNS-Sequenz Sequenz ID-No: 2 ist, funktionell mit dem Strukturgen eines zu exprimierenden, heterologen Proteins verknüpft ist.

7. Mikroorganismen enthaltend mindestens eine Expressionskassette gemäß Anspruch 6.

8. Mikroorganismen enthaltend mindestens ein Expressionssystem gemäß Anspruch 1.

9. Fermentationsverfahren zur Produktion von Proteinen, mittels Mikroorganismen, dadurch gekennzeichnet, daß Mikroorganismen gemäß Anspruch 7 oder 8 verwendet werden.

10. Verfahren zur Herstellung eines Mikroorganismus gemäß Anspruch 8, dadurch gekennzeichnet, daß in einen beliebigen Mikroorganismus gegebenenfalls mindestens ein Gen für ein Regulatorprotein gemäß Anspruch 4 oder 5 und/oder gegebenenfalls mindestens eine Expressionskassette gemäß Anspruch 7 eingebracht werden.

11. Verwendung von Fnr - negativen Mikroorganismen als Wirtsstamm für ein Expressionssystem gemäß Anspruch 1.

## Claims

1. Expression system which can be regulated by acetate, pH and oxygen, which expression system comprises a trans-acting regulator protein and a promoter which can be activated by this protein, characterized in that the regulator protein encompasses an amino acid sequence which is at least 75% homologous with the amino acid sequence Seq. ID-No:1, and the promoter encompasses a DNA sequence which is at least 95% homologous with the bases 315 to 397 of the DNA sequence Seq. ID-No:2.

2. Expression system according to Claim 1, characterized in that it maximally expresses any desired structural genes under the control of the expression system at an oxygen partial pressure, pO₂, of 0-5% and a pH of 6.0-6.5 and in the presence of acetate at a concentration of 40-60 mM

3. Regulator protein, characterized in that it brings about optimal activation of the bud promoter from Klebsiella terrigena (DSM2687) in association with oxygen limitation and in the presence of acetate and at a pH of the culture medium of from pH 6.0 to pH 6.5.

4. Regulator protein according to Claim 3, characterized in that it encompasses an amino acid sequence which is at least 75% homologous with the amino acid sequence Seq. ID-No.1.

5. Gene which encodes a protein according to Claim 3 or 4.

6. Expression cassette, wherein a promoter, which encompasses a DNA sequence which is at least 95% homologous with bases 315 to 397 in the DNA sequence Sequence ID-No: 2, is functionally linked to the structural gene of a heterologous protein which is to be expressed.

7. Microorganism which harbours at least one expression cassette according to Claim 6.

8. Microorganism which harbours at least one expression system according to Claim 1.

9. Fermentation process for producing proteins by means of microorganisms, characterized in that microorganisms according to Claim 7 or 8 are used.

10. Process for preparing a microorganism according to Claim 8, characterized in that, where appropriate, at least one gene for a regulator protein according to Claim 4 or 5 and/or, where appropriate, at least one expression cassette according to Claim 7 are introduced into any desired microorganism.

11. Use of an Fnr-negative microorganism as a host strain for an expression system according to Claim 1.

## Revendications

1. Système d'expression régulable par l'acétate, la valeur du pH et l'oxygène, qui comprend une protéine régulatrice agissant en trans et un promoteur activable par cette protéine, caractérisé en ce que la protéine régulatrice comprend une séquence d'acides aminés qui est homologue à au moins 75% avec la séquence d'acides aminés SEQ ID-No:1, et en ce que le promoteur comprend une séquence d'ADN qui est homologue à au moins 95% avec les bases 315 à 397 de la séquence d'ADN SEQ ID-No:2.

2. Système d'expression suivant la revendication 1, caractérisé en ce qu'il exprime de manière maximale n'importe quel gène de structure sous le contrôle du système d'expression à une pression partielle en oxygène pO₂ de 0 à 5%, une valeur du pH de 6,0 à 6,5 et en présence d'acétate à une concentration de 40 à 60 mM.

3. Protéine régulatrice, caractérisée en ce qu'elle provoque une activation optimale du promoteur bud de Klebsiella terrigena (DSM2687) avec une limitation de l'oxygène et en présence d'acétate et à une valeur du pH du milieu de culture de pH 6,0 à pH 6,5.

4. Protéine régulatrice suivant la revendication 3, caractérisée en ce qu'elle comprend une séquence d'acides aminés qui est homologue à au moins 75% avec la séquence d'acides aminés SEQ ID-No:1.

5. Gène codant pour une protéine suivant l'une quelconque des revendications 3 ou 4.

6. Cassette d'expression, caractérisée en ce qu'un promoteur, qui contient une séquence d'ADN qui est homologue à au moins 95% avec les bases 315 à 397 de la séquence d'ADN SEQ ID-No:2, est fonctionnellement lié au gène de structure d'une protéine hétérologue à exprimer.

7. Micro-organismes contenant au moins une cassette d'expression suivant la revendication 6.

8. Micro-organismes contenant au moins un système d'expression suivant la revendication 1.

9. Procédé de fermentation pour la production de protéines, au moyen de micro-organismes, caractérisé en ce que l'on utilise des micro-organismes suivant l'une quelconque des revendications 7 ou 8.

10. Procédé de préparation d'un micro-organisme suivant la revendication 8, caractérisé en ce que, le cas échéant, au moins un gène d'une protéine régulatrice suivant l'une quelconque des revendications 4 ou 5 et/ou, le cas échéant, au moins une cassette d'expression suivant la revendication 7 sont introduits dans un micro-organisme quelconque.

11. Utilisation de micro-organismes Fnr-négatifs comme souche hôte pour un système d'expression suivant la revendication 1.


### SEQUENZPROTOKOLL

### (1) ALLGEMEINE ANGABEN:

(i) ANMELDER:
   (A) NAME: Consortium fuer elektrochemische Industrie GmbH
   (B) STRASSE: Zielstattstr. 20
   (C) ORT: Muenchen
   (D) BUNDESLAND: Bayern
   (E) LAND: Deutschland
   (F) POSTLEITZAHL: 81379
   (G) TELEFON: 089/74844-0
   (H) TELEFAX: 089/74844-350
   (I) TELEX: 5215553 cons d
(ii) BEZEICHNUNG DER ERFINDUNG: Steuerbares Expressionssystem
(iii) ANZAHL DER SEQUENZEN: 15
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)

### (2) ANGABEN ZU SEQ ID NO: 1:

(i) SEQUENZKENNZEICHEN:
   (A) LÄNGE: 290 Aminosäuren
   (B) ART: Aminosäure
   (C) STRANGFORM:
   (D) TOPOLOGIE: linear
(ii) ART DES MOLEKÜLS: Protein
(vi) URSPRÜNLICHE HERKUNFT:
   (A) ORGANISMUS: Klebsiella terrigena
   (B) STAMM: DSM2867
(xi) SEQUENZBESCHREIBUNG: SEQ ID NO: 1:
   Met Glu Leu Arg Tyr Leu Arg Tyr Phe Val Ala Val Ala Glu Ala Arg
   1 5 10 15
   Asn Phe Thr Arg Ala Ala His Asp Leu Gly Ile Ser Gln Pro Pro Leu
   20 25 30
   Ser Gln Gln Ile Gln Arg Leu Glu Arg Glu Ile Gly Thr Pro Leu Leu
   35 40 45
   Arg Arg Leu Thr Arg Gly Val Glu Leu Thr Glu Ala Gly Glu Ser Phe
   50 55 60
   Tyr Val Asp Ala Cys Gln Ile Leu Ala Leu Ser Asp Ala Ala Leu Glu
   65 70 75 80
   Lys Thr Lys Gly Ile Ala Arg Gly Met Asn Gly Ser Leu Val Pro Gly
   85 90 95
   Ile Thr Ser Ser Ala Ala Phe His Ser Gln Ile Phe Ser Leu Leu Tyr
   100 105 110
   Gln Phe Gln Gln Arg Tyr Pro Ala Val Ala Leu Arg Gln Val Glu Gly
   115 120 125
   Asn Met Ala Thr Leu Met His Ala Leu Gly Glu Ala Glu Leu Asp Ile
   130 135 140
   Ala Phe Val Arg Leu Pro Cys Glu Ser Ser Lys Ala Phe Asn Leu Arg
   145 150 155 160
   Ile Ile Ala Glu Glu Pro Met Val Ile Ala Leu His Arg Ser His Pro
   165 170 175
   Leu Ser Gly Glu Ser Ala Leu Ser Leu Ala Gln Leu Ser Asp Ala Val
               180                 185                 190
   Pro Val Ile Phe Pro Pro Glu Val Ala Pro Gly Leu Tyr Glu Gln Val
           195                 200                 205
   Tyr Asp Gly Cys Arg Arg Ala Gly Val Asp Met Ser Arg Ala Arg Gln
       210                 215                 220
   Ser Ser Gln Ile Ser Ser Ser Ile Ser Met Val Asp Ala Gly Phe Gly
   225                 230                 235                 240
   Phe Ala Leu Val Pro Gln Ser Met Thr Cys Ile Cys Leu Pro Asn Val
                   245                 250                 255
   Thr Trp His Pro Leu Gln Asp Ala Ser Leu Lys Thr Glu Ile Ala Ile
               260                 265                 270
   Ala Trp Arg Arg Phe Glu Arg Ser Arg Thr Val Lys Arg Phe Leu Glu
           275                 280                 285
   Met Phe
       290

### (2) ANGABEN ZU SEQ ID NO: 2:

(i) SEQUENZKENNZEICHEN:
   (A) LÄNGE: 1453 Basenpaare
   (B) ART: Nucleotid
   (C) STRANGFORM: Doppelstrang
   (D) TOPOLOGIE: linear
(ii) ART DES MOLEKÜLS: Genom-DNA
(vi) URSPRÜNLICHE HERKUNFT:
   (A) ORGANISMUS: Klebsiella terrigena
   (B) STAMM: DSM2687
(vii) UNMITTELBARE HERKUNFT:
   (B) CLON(E): pBAK14/16
(viii) POSITION IM GENOM:
   (C) EINHEITEN: bp
(xi) SEQUENZBESCHREIBUNG: SEQ ID NO: 2:
   GGATCCGCCC GGGCTTTACG CGCGCTGCCG TCAGCGCGCA GCTGGTAGAC CTCGCTGCTA   60
   AAGGCAATCA GTTCGCCATC GAGTTCGTTA AAGGTGCCGA GACCGAAGTC GCCGTGGGTC  120
   AGCAGGTCGG CGATGGTGGT GCTACCCTCA TAGACCCCGC TCAGCAGCGC GCTCATCAGA  180
   GAGGTCTGAT AGATAACGCT ATCAGGGTGG TGGGCGGAGA AGCCGCGTAC GGTTTCGCAC  240
   AGGCTCTCCT GGCAGGTGCA TTCAGGATAA TGATTCACAA TCCACTTCCT CGTTCAACAA  300
   ATATAAGAAA GATTAAATAA ATATTGACCC GATTCAGCTC TCAGTTCCAA TATAGAATCC  360
   ATGCTGGTTT GAGACGTTTA CGATATGGAA CTTCGCTATT TACGTTATTT TGTCGCCGTT  420
   GCCGAGGCGC GGAACTTCAC CCGGGCGGCC CACGATCTTG GCATTTCTCA ACCGCCACTA  480
   AGTCAGCAAA TTCAGCGACT TGAGCGAGAA ATAGGGACTC CGCTGCTGCG TCGTTTGACG  540
   CGGGGGGTTG AGCTGACGGA GGCCGGAGAG TCGTTCTACG TCGACGCGTG TCAGATCCTC  600
   GCCTTAAGCG ATGCGGCGCT GGAAAAAACC AAGGGGATTG CGCGGGGCAT GAACGGTAGC  660
   CTGGTGCCGG GGATCACCAG TTCAGCTGCT TTTCATTCGC AGATTTTCTC TTTGCTGTAC  720
   CAGTTTCAGC AGCGCTATCC GGCGGTGGCT CTGCGCCAGG TCGAAGGCAA TATGGCGACG  780
   CTGATGCATG CCCTGGGCGA GGCGGAGCTG GATATCGCCT TTGTGCGCCT GCCGTGTGAA  840
   AGCAGCAAGG CGTTTAATTT GCGCATTATT GCCGAGGAGC CGATGGTTAT CGCGCTGCAT  900
   CGCTCGCACC CGCTCTCCGG GGAAAGTGCG CTCTCTCTGG CGCAGCTGAG CGACGCGGTG 960
   CCGGTTATTT TCCCGCCGGA GGTGGCGCCG GGCCTCTACG AGCAGGTTTA TGATGGCTGT 1020
   CGGCGTGCCG GGGTCGATAT GAGCCGCGCC AGGCAATCTT CACAGATCTC GTCTTCTATT 1080
   AGCATGGTGG ACGCGGGCTT CGGCTTTGCG CTGGTGCCTC AGTCGATGAC CTGTATCTGC 1140
   CTTCCCAACG TCACATGGCA TCCCTTGCAG GACGCGTCGC TGAAGACGGA GATCGCCATC 1200
   GCGTGGCGGC GTTTTGAACG TTCGCGGACG GTAAAGCGTT TTCTGGAGAT GTTTTAGGCG 1260
   GGGCGCAGGG CTAGCAGGTA TAGACGTTTG CCGCGGTTGG CCCGCGCAGG CCGTTAACCC 1320
   GACGAAACTC AACGGAATAC CGGGCGTCAT CGCCGTGGAC TCGTTGGGGG ATAATGCGGA 1380
   AATATGAACC TGAACGTCTT TACGACCGTC GGAAGGGACG ATAAGGCCTC TGCCGGTTTT 1440
   ATTATCAAAG CTT 1453

### (2) ANGABEN ZU SEQ ID NO: 3:

(i) SEQUENZKENNZEICHEN:
   (A) LANGE: 39 Basenpaare
   (B) ART: Nucleotid
   (C) STRANGFORM: Doppelstrang
   (D) TOPOLOGIE: linear
(ii) ART DES MOLEKÜLS: Genom-DNA
(vii) UNMITTELBARE HERKUNFT:
   (B) CLON(E): pBTL142 und pRBL2
(viii) POSITION IM GENOM:
   (C) EINHEITEN: bp
(xi) SEQUENZBESCHREIBUNG: SEQ ID NO: 3:
   TATCCTGAAT GCACCTGCCA GGATCCCGTC GTATTACAA 39

### (2) ANGABEN ZU SEQ ID NO: 4:

(i) SEQUENZKENNZEICHEN:
   (A) LÄNGE: 13 Aminosäuren
   (B) ART: Aminosäure
   (C) STRANGFORM:
   (D) TOPOLOGIE: linear
(ii) ART DES MOLEKÜLS: Peptid
(v) ART DES FRAGMENTS: inneres Fragment
(vii) UNMITTELBARE HERKUNFT:
   (B) CLON(E): pBTL142 und pRBL2
(xi) SEQUENZBESCHREIBUNG: SEQ ID NO: 4:
   Tyr Pro Glu Cys Thr Cys Gln Asp Pro Val Val Leu Gln
   1               5                   10

### (2) ANGABEN ZU SEQ ID NO: 5:

(i) SEQUENZKENNZEICHEN:
   (A) LÄNGE: 29 Basenpaare
   (B) ART: Nucleotid
   (C) STRANGFORM: Doppelstrang
   (D) TOPOLOGIE: linear
(ii) ART DES MOLEKÜLS: Genom-DNA
(vii) UNMITTELBARE HERKUNFT:
   (B) CLON(E): pBUD100
(viii) POSITION IM GENOM:
   (C) EINHEITEN: bp
(xi) SEQUENZBESCHREIBUNG: SEQ ID NO: 5:
   CGAGGAAGTG GACCATGGAA AGAAACCGT                                        29

### (2) ANGABEN ZU SEQ ID NO: 6:

(i) SEQUENZKENNZEICHEN:
   (A) LÄNGE: 29 Basenpaare
   (B) ART: Nucleotid
   (C) STRANGFORM: Doppelstrang
   (D) TOPOLOGIE: linear
(ii) ART DES MOLEKÜLS: Genom-DNA
(vii) UNMITTELBARE HERKUNFT:
   (B) CLON(E): pBUD200
(viii) POSITION IM GENOM:
   (C) EINHEITEN: bp
(xi) SEQUENZBESCHREIBUNG: SEQ ID NO: 6:
   CGAGGAAGTG GATTATGAAA AGAAACCGT 29

### (2) ANGABEN ZU SEQ ID NO: 7:

(i) SEQUENZKENNZEICHEN:
   (A) LÄNGE: 25 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligol
(viii) POSITION IM GENOM:
   (C) EINHEITEN: bp
(xi) SEQUENZBESCHREIBUNG: SEQ ID NO: 7:
   GGGGATCCTG GCAGGTGCAT TCAGG 25

### (2) ANGABEN ZU SEQ ID NO: 8:

(i) SEQUENZKENNZEICHEN:
   (A) LÄNGE: 25 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo2
(viii) POSITION IM GENOM:
   (C) EINHEITEN: bp
(xi) SEQUENZBESCHREIBUNG: SEQ ID NO: 8:
   GGGGATCCAT CGTGGGCCGC CCGAG 25

### (2) ANGABEN ZU SEQ ID NO: 9:

(i) SEQUENZKENNZEICHEN:
   (A) LÄNGE: 33 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo3
(xi) SEQUENZBESCHREIBUNG: SEQ ID NO: 9:
   GGGGATCCCG CCTAAAACAT CTCCAGAAAA CGC 33

### (2) ANGABEN ZU SEQ ID NO: 10:

(i) SEQUENZKENNZEICHEN:
   (A) LÄNGE: 10 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo4
(viii) POSITION IM GENOM:
   (C) EINHEITEN: bp
(xi) SEQUENZBESCHREIBUNG: SEQ ID NO: 10:
   CGGAATTCCG 10

### (2) ANGABEN ZU SEQ ID NO: 11:

(i) SEQUENZKENNZEICHEN:
   (A) LÄNGE: 43 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): OligoS
(viii) POSITION IM GENOM:
   (C) EINHEITEN: bp
(xi) SEQUENZBESCHREIBUNG: SEQ ID NO: 11:
   TCTAGAAGCT TCGCGACCAA CCGCGGCAAA CGTCTATACC TGC 43

### (2) ANGABEN ZU SEQ ID NO: 12:

(i) SEQUENZKENNZEICHEN:
   (A) LÄNGE: 37 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo6
(viii) POSITION IM GENOM:
   (C) EINHEITEN: bp
(xi) SEQUENZBESCHREIBUNG: SEQ ID NO: 12:
   TCTAGAAGCT TCCATGGTCC ACTTCCTCGT TCAACAA 37

### (2) ANGABEN ZU SEQ ID NO: 13:

(i) SEQUENZKENNZEICHEN:
   (A) LÄNGE: 40 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo7
(viii) POSITION IM GENOM:
   (C) EINHEITEN: bp
(xi) SEQUENZBESCHREIBUNG: SEQ ID NO: 13:
   TCTAGAAGCT TCCATGGAAA GAAACCGTTT TTTTAATACC 40

### (2) ANGABEN ZU SEQ ID NO: 14:

(i) SEQUENZKENNZEICHEN:
   (A) LÄNGE: 41 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo8
(viii) POSITION IM GENOM:
   (C) EINHEITEN: bp
(xi) SEQUENZBESCHREIBUNG: SEQ ID NO: 14:
   TCTAGAAGCT TGAATTCTTA AAACGAGCCA TTCGTTGTTT G                41

### (2) ANGABEN ZU SEQ ID NO: 15:

(i) SEQUENZKENNZEICHEN:
   (A) LÄNGE: 36 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Synthetische DNS"
(vii) UNMITTELBARE HERKUNFT:
   (B) CLON(E): Oligo9
(viii) POSITION IM GENOM:
   (C) EINHEITEN: bp
(xi) SEQUENZBESCHREIBUNG: SEQ ID NO: 15:
   GAACGAGGCC GTGGATTATG AAAAGAAACC GTTTTT                      36